# EUROPEAN PATENT SPECIFICATION

(11) **EP 1 830 705 B1**
(45) Date of publication and mention of the grant of the patent: **29.12.2010**
(21) Application number: 05807215.8
(22) Date of filing: 28.11.2005
(51) Int. Cl.: A61B 5/103, A61B 5/00

(54) **A METHOD OF OBSERVING BIOLOGICAL TISSUE, IN PARTICULAR HUMAN SKIN**
VERFAHREN ZUR BEOBACHTUNG VON BIOLOGISCHEM GEWEBE, INSBESONDERE MENSCHLICHER HAUT
PROCEDE D'OBSERVATION DE TISSU BIOLOGIQUE, NOTAMMENT LA PEAU HUMAINE

(30) Priority: 26.11.2004 FR 0412581; 31.01.2005 US 647764 P
(43) Date of publication of application: 12.09.2007
(73) Proprietor: L'Oréal, 75008 Paris (FR)
(72) Inventor: LEVEQUE, Jean-Luc, F-75017 PARIS (FR); SOJIC, Neso, F-33610 CESTAS (FR)
(74) Representative: Tanty, François
(86) International application number: PCT/IB2005/053927
(87) International publication number: WO 2006/056965

(56) References cited:
- US-A- 5 893 364
- US-A1- 2004 042 013
- US-A1- 2004 125 996
- US-B1- 6 177 984
- US-B1- 6 411 838

## Description

The present invention relates to observing biological tissue, and more particularly, but not exclusively, to observing human skin.

US 5 893 364 discloses a measuring head for light reflexion measurements comprising light passages for the light irradiate into the test object and separate light passages for the light leaving the test object. No image of the test object is obtained.

US 6 411 838 discloses an optical system for the examination of samples comprising transmitter and receiver portions , no single optical component is used in both the transmitter and the receiver portions of the device. Such a system necessitates special separation means to prevent interference from scattered light.

WO 2004/026125 discloses an endoscope for viewing an object located in an individual's body. The endoscope comprises an illumination fiber to illuminate an object to be viewed and a coherent fiber bundle to relay an image of the objet to the proximal end of the endoscope for viewing.

US 6741884 discloses a balloon probe comprising an imaging collection fiber bundle adapted to obtain spectroscopic information useful for the diagnosis and treatment of disease.

WO 02/069784 discloses an apparatus for measuring spectral and temporal fluorescence properties of tissue or cells for diagnostic purposes.

WO 2004/043255 discloses a method for assessing the efficacity of olein care products and discloses among others the use of polarized light photography for taking images dominated by the light reflected from corneocytes.

It has been known for many years that the size of corneocytes at the surface of the skin is directly related to their transit speed in the epidermis.

In young individuals, the cells are small because the level of metabolism in the epidermis is high and the transit time is short. The opposite applies in old people.

Measuring the size of corneocytes can therefore provide useful information, in particular for cosmetics.

Currently, this measurement can be carried out *ex vivo*, with the cells being collected by scraping or by exposing the skin to a detergent solution, and then being dyed and placed under a microscope. Their size is determined by image processing, with at least one thousand cells preferably being used in order to determine the mean size with accuracy.

There exists another possibility whereby an adhesive is applied to the skin, said adhesive taking the surface cells with it when removed. The surface cells are dyed, and then measured under a microscope.

*In vivo*, the corneocytes are observed by means of a confocal microscope, after the skin has been dyed.

A confocal microscope is an instrument that is costly and complex to use, and in addition, the number of corneocytes present in the observed field is relative small, thereby requiring numerous regions to be observed in order to determine the mean size of the corneocytes.

The invention seeks to propose a noel means of enabling corneocytes to be observed, for example *in vivo*.

In one of its aspects, the invention provides a method of observing biological tissue, the method being defined by appended claim 1.

In exemplary embodiments, the first end of the bundle of optical fibers is oriented in a direction substantially perpendicular to the surface of the tissue.

The first end of the bundle may be put into contact with the surface of the tissue.

In a variant, the first end of the bundle may be spaced from the surface of the tissue by a distance.

The invention may be configured to obtain an image of the surface of the tissue that can be useful, e.g. for skin, for determining the mean size of corneocytes, without any need for complex equipment, such as a confocal microscope.

Light may be collected by the first end of the bundle through an optical system comprising at least one lens.

The use of such an optical system may make it possible to increase the spatial resolution and to minimize a pixelisation effect.

In exemplary embodiments, the distance between the optical system and the surface of the tissue may range from 100 µm to 1 mm, for example may range from 200 µm to 500 µm.

The at least one lens of the optical system may comprise a magnifying lens and/or a collecting lens.

The optical system may comprise a first lens and second lens, light from the surface of the tissue passing through the second lens and then the first lens. The first lens may be a collimating lens and the second lens may be magnifying lens.

In exemplary embodiments, the length of the first lens may range from 4.4 to 6.8 mm. The length of the second lens may range from 1.8 to 2.9 mm.

The at least one lens of the optical system may comprise a lens with an index gradient. This lens may have a g value of the index gradient at 670 nm greater than or equal to 0.25 mm⁻¹.

The at least one lens of the optical system may be cylindrical and may have an external diameter less than or equal to 2 mm. Both the first and second lens may be cylindrical.

The size of the fibers used determines the resolution of the image. The spatial resolution may be better than 5 micrometers (µm), preferably better than 4 µm, and more preferably better than 3 µm. The optical fibers may present a diameter that is less than or equal to 4 µm, better less than or equal to 3 µm, e.g. that lies in the range from about 2 µm to about 3 µm.

Using an optical system as defined above may provide a resolution better than 2 µm, or even better than 1.5 µm.

The relative positions of the fibers within the bundle may be the same at the inlet and at the outlet of said bundle. Each fiber may correspond to one pixel of the image.

The number of optical fibers in the bundle may be greater than or equal to 2500, being preferably greater than or equal to 5000. The number of optical fibers in the bundle may thus be greater than or equal to 7500, and better greater than or equal to 10,000.

The section occupied by the bundle of optical fibers may be greater than or equal to 0.01 square millimeters (mm²), better greater than or equal to 0.05 mm², better still greater than or equal to 0.07 mm², e.g. greater than or equal to 0.1 mm², or even several tenths of a mm² or greater, e.g. 1 mm² or several mm².

The tissue is skin or hair. The observation may be an *in vivo* or an *in vitro* observation.

The method includes image processing so as to determine information associated with corneocytes, said information relating to the number of corneocytes present in the image and/or to their mean or median size, for example.

Prior to making the observation, a fluorescent marker may be put into contact with the tissue.

In the presence of the fluorescent marker, light centered on a first wavelength may be injected into the fibers, and the observation may be made at a second wavelength that is different from the first. By way of example, the wavelengths may be selected as a function of the fluorescent marker, so as to maximize contrast in the obtained image.

The image may be acquired with an array sensor, for example a CCD sensor.

At least two observations may be made at two different locations of the tissue. Two observations may be made at two respective regions that have been exposed in different ways to a given environment, e.g. two regions that have been exposed in different ways to ultraviolet light and/or two regions that have been treated differently with at least one substance. A plurality of observations may be made at different locations, and the results of the various observations may be processed statistically to obtain a value that is representative of an observed parameter, for example a mean or median value.

Information relating to aging of the skin may be delivered as a function of the number of observed corneocytes and/or as a function of their mean or median size, for example.

The observed surface may be situated outside or inside a hair follicle.

The result of an observation may be compared with reference data, which may come from the observed person or from a reference population.

By way of example, it is possible to compare information resulting from an observation made during a first time interval with information resulting from an observation made during a second time interval that is different from the first. By way of example, this can make it possible to establish a diagnosis or a prognosis, or to determine the effect of a substance or of a treatment.

In another of its aspects, the invention also provides a method of revealing the effect of a treatment, for example a non-therapeutic treatment, the method comprising:
· before the treatment, making a first observation by means of the method as defined above;
· after the treatment, making a second observation by means of the same method; and
· comparing the results of the first and second observations, and optionally deducing therefrom information relating to at least one effect of the treatment.

By way of example, the treatment may comprise applying a cosmetic or a skin-care product, or it may comprise taking food supplements or medication.

In another of its aspects, the invention also provides a method of predicting changes that will occur in a physical or biological parameter of some tissue, e.g. the size of its corneocytes, its apparent age, ..., the method comprising:
· making at least one observation by implementing the method as defined above; and
· from the result of the observation, predicting changes that will occur in said parameter.

In another of its aspects, the invention also provides a method of prescribing a substance, for example a cosmetic substance, the method comprising:
· making at least one observation by implementing the method as defined above; and
· from the result of the observation, prescribing at least one substance, for example one cosmetic substance.

In another of its aspects, the invention also provides a method of observing biological tissue, the method comprising:
· putting a first end of the fibers of a bundle of optical fibers into contact with a surface of the tissue, to observe an image at a second end of the fibers of the bundle.

In another of its aspects, the invention also provides a method of observing corneocytes comprising:
· illuminating at a first wavelength skin,
· observing an image of the corneocytes at a second wavelength different form the first, using a bundle of optical fibers, the image being observed at a second end of the bundle while the first end receives light from the skin.

The skin illumination may be obtained by injecting light into the second end of the bundle or otherwise.

In another of its aspects, the invention also provides a method of observing a surface of the tissue through an optical system assembled to a first end of a bundle of optical fibers so as to produce at a second end thereof an image.

The image may be observed through a lens, for example a microscope lens.

Light may be injected in the second end of the bundle to illuminate the surface of the tissue.

In another of its aspects, the invention also provides skin-imaging apparatus defined by claim 48.

The processor system may be arranged to deliver information automatically.

The resolution of said bundle may be sufficient for allowing the obtained image to show corneocytes present at the surface of the skin.

The first end of the bundle may be configured for contacting skin.

The first end of the bundle may be configured for observing skin while being spaced from the surface of the tissue by a distance.

The apparatus may further comprises an optical system comprising at least one lens.

The at least one lens may comprise at least one of a magnifying lens and a collimating lens.

The optical system may comprise a first lens and second lens.

The first lens may be a collimating lens and the second lens may be a magnifying lens, light from the surface of the tissue passing through the second lens and then the first lens.

The length of the first lens may range from 4.4 to 6.8 mm and the length of the second lens may range from 1.8 to 2.9 mm.

The at least one lens of the optical system may comprise a lens with an index gradient, the g value of the index gradient at 670 nm may be greater or equal to 0.25 mm-1. The distance from which the optical system is spaced from the surface of the tissue during observation may range from 100 µm to 1 mm, for example from 200 µm to 500 µm.

The apparatus includes a dichroic mirror, a light source, and a camera, the dichroic mirror reflecting, into the bundle of fibers, the light emitted by the light source, and the camera observing the image that is reflected back via the fibers, after passing through the dichroic mirror.

The apparatus includes a monochromatic filter or a monochromator associated with the light source. The apparatus also includes a monochromatic filter or a monochromator associated with the camera.

The invention is not limited to a particular light source, and said light source may comprise an incandescent lamp, a light emitting diode (LED), a laser, or a discharge lamp (xenon, mercury, ...).

The apparatus may include a recorder system enabling images to be stored. By way of example, this can enable them to be compared with other images coming from the same person or from a reference population, for example.

The relative positions of the fibers within the bundle may be the same at the inlet and at the outlet of said bundle.

In another of its aspects, the invention also provides a kit comprising:
· apparatus as defined above; and
· a receptacle containing a fluorescent marker for applying to the skin before it is observed.

The invention also provides a method of promoting a treatment and/or the sale of a substance, which method puts forward an effect of the substance, as revealed by an observation method as defined above. The promotion could be carried out using any sales channel.

The invention can be better understood on reading the following detailed description of non-limiting embodiments thereof, and on examining the accompanying drawings, in which:
· Figure 1 is a diagrammatic view of imaging apparatus according to an exemplary embodiment of the invention;
· Figure 2 is a diagrammatic and fragmentary cross-section of the bundle of optical fibers in Figure 1;
· Figure 3 is a diagrammatic view showing the arrangement of various optical components of the Figure 1 apparatus;
· Figure 4 shows the possibility of connecting the Figure 1 apparatus to a remote server;
· Figure 5 is an example of an image that can be observed using the Figure 1 apparatus;
· Figures 6 to 10 are block diagrams showing examples of methods of the invention; and
· Figure 11 shows the possibility of adding to the apparatus of Figure 3 an optical system.

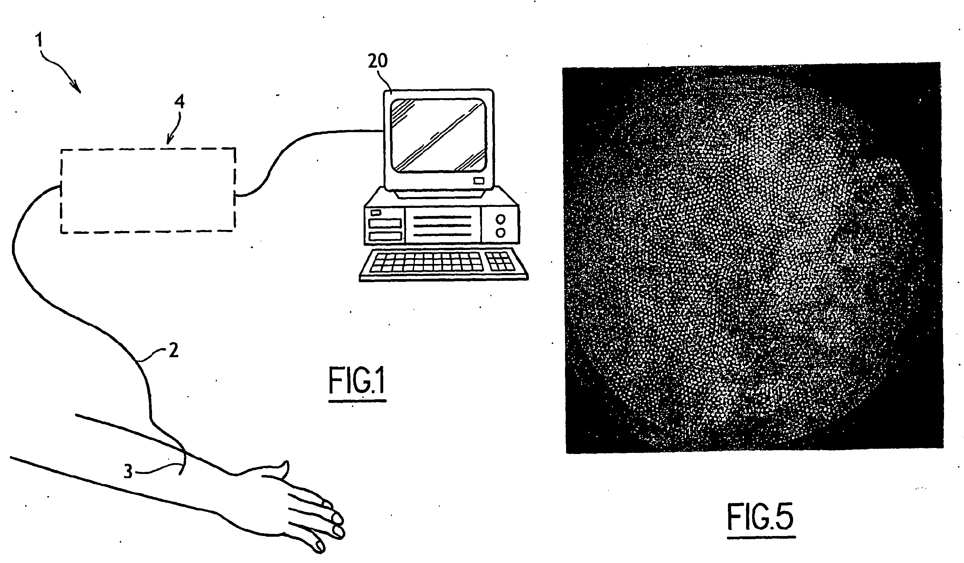

Figure 1 shows apparatus 1 in accordance with the invention and including a bundle 2 of optical fibers having a first end 3 that may for example be put directly into contact with an observed surface, e.g. a skin surface, as shown, without any need for intermediate optics.

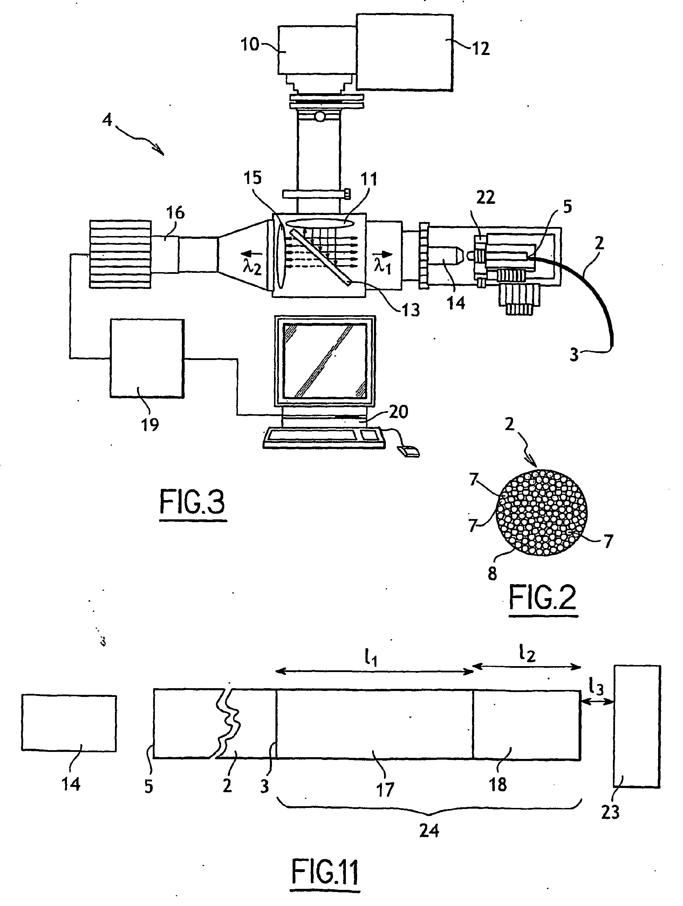

The other end 5 of the bundle 2 is connected to a device 4 which is described in greater detail below, with reference to Figure 3.

The optical fibers 7 of the bundle 2 extend parallel to one another, and may be of circular cross-section, as shown in Figure 2. The fibers 7 may be contained in a sheath 8 which may also be of circular section.

In the embodiment under consideration, the disposition of the fibers 7 relative to one another at the inlet of the bundle is the same as their relative disposition at the outlet of the bundle, with each fiber thus constituting a kind of pixel in the image.

In the embodiment under consideration, the fibers 7 are substantially identical, e.g. being made of glass, and being of diameter that is less than or equal to 4 µm, with the number of fibers 7 being greater than or equal to 10,000, for example, and with the total section occupied by the fibers 7 of the bundle being greater than 0.5 mm², for example. For the purposes of clarity, the drawing shows only some of the fibers, without complying with relative proportions.

The size and the number of fibers can be selected as a function of the desired resolution.

As can be seen in Figure 3, the device 4 comprises a light source 10, e.g. an incandescent lamp or a discharge lamp and its power supply 12, a first monochromatic filter 11 enabling the light emitted by the source 10 to be filtered around a first wavelength λ₁, and a dichroic mirror 13 enabling a fraction of the light coming from the filter 11 to be reflected towards a lens 14 that is arranged to inject the light into the second end 5 of the bundle 2. The lens 14 may be a microscope lens.

The apparatus 1 may comprise a micro-positioner 22 to facilitate placing the bundle 2 substantially in front of the lens 14.

Part of the light that is reflected back into the bundle 2 at the first end 3 passes through the dichroic mirror 13, and then through a second optical filter 15 centered on a second wavelength λ₂ that is different from the first, so as to be observed by an image-analyzer system including an array sensor 16, e.g. a CCD sensor and a CCD controller 19 sending data to an image processor system 20. By way of example, the image-analyzer system is a digital camera.

In a variant, the filters 11 and 15 are replaced by monochromators.

In the embodiment under consideration, the dichroic mirror 13 presents a plane that is at an angle of substantially 45° to the incident light emitted by the source 10, and the light that is returned by the lens 14 is along a direction that is substantially perpendicular to the light emitted by the source 10.

Naturally, various modifications can be applied to the device 4 without going beyond the ambit of the present invention.

For example, the source 10 that is used can be substantially monochromatic, e.g. by using LEDs or a laser. Where appropriate, the sensor 16 can be wavelength selective, and not require the presence of the second filter 15. When the source 10 is monochromatic, the first filter 11 can be eliminated.

In the exemplary embodiment of Figure 1, the first end of the bundle may be put directly into contact with a surface to be observed.

However, it is also possible, according to another exemplary embodiment, to observe the surface of the tissue at a distance though an optical system 24 assembled to the first end of the bundle 2 of fibers, as shown in figure 11. The device 4 may be as described above. The optical system 24 may be spaced from the surface of the tissue 23 during observation by a distance 1₃, for example about 300 µm.

In the embodiment of Figure 11, the optical system 24 is fixed to the bundle 2, with a glue transparent to visible light, but the optical system 24 may be fixed differently for example using an external sleeve.

The optical system 24 may comprise a proximal or first lens 17 and a distal or second lens 18.

The first lens 17 may be a collimating lens and the second lens 18 may be a magnifying lens. The first lens 17 may collimate the light beam outing from the bundle and the second lens 18 may re-focus the light beam towards the surface to be observed.

The lenses 17 and 18 may be cylindrical.

In an exemplary embodiment, the characteristics of the first lens may be:
- numerical aperture = 0.2
- pitch = 0.25
- central refractive index at 670 nm = 1.5297
- g value of the index gradient at 670 nm = 0.255 mm⁻¹
- external diameter = 1 mm
- length l₁ = 5.5 mm

The characteristics of the second lens may be:
- numerical aperture = 0.5
- pitch = 0.2
- central refractive index at 670 nm = 1.6289
- g value of the index gradient at 670 nm = 0.654 mm⁻¹
- external diameter = 1 mm
- length l₂ = 2.3 mm

With such an optical system, the spatial resolution may improve by a 2.7 factor, from 3 µm to 1.1 µm.

The image processor system 20 may be in the form of a micro-computer.

The micro-computer can be programmed to analyze at least one image observed by the array sensor 16.

When the observed surface is a human skin surface, the image observed by the array sensor 16 can be in the form shown in Figure 5, for example, in which the outlines of the corneocytes present at the surface of the skin can be seen.

The image-processor system 20 can be arranged to calculate a mean or median size of corneocytes either from a single image or from a plurality of images and by performing statistical processing thereon, with the various images being observed after moving the end 3 over the skin, for example.

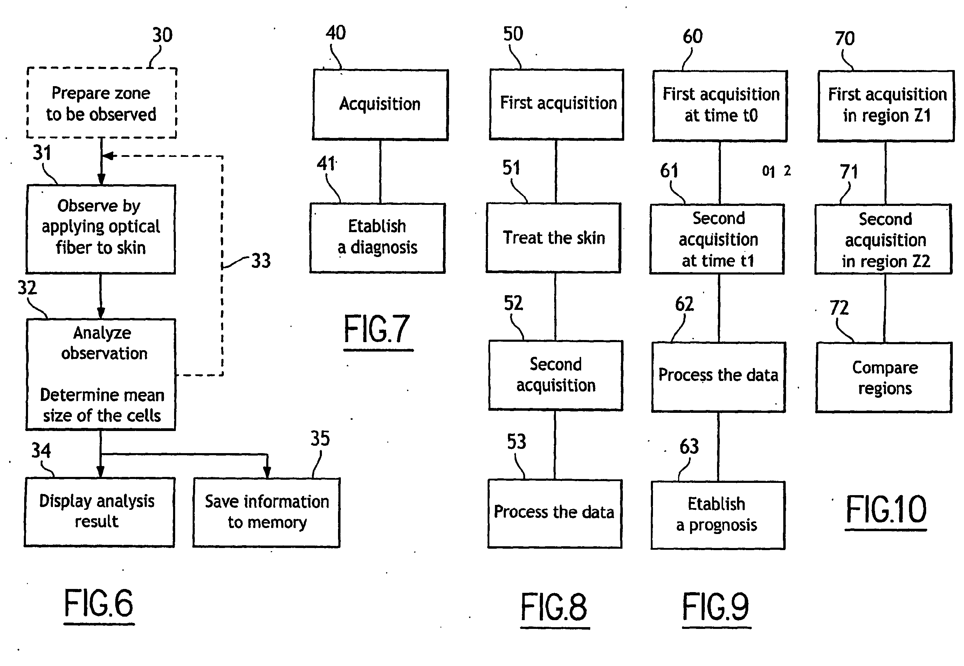

As shown in Figure 6, the apparatus 1 can be used as follows:

In a first step 30, the observed surface can be prepared for observation.

For human skin, preparation can comprise applying a fluorescent marker to the skin, e.g. a solution of fluorescein or fluorescein sodium.

The skin receiving light at the wavelength λ₁ can re-emit light at a wavelength λ₂ because of the presence of the fluorescent marker whose concentration varies as a function of the disposition of the cells, and in particular causes the outlines of said cells to appear. For fluorescein, the wavelength that is absorbed is about 493.5 nanometers (nm), so λ₁ is set to be close to that value. The wavelength that is emitted is about 520 nm, and λ₂ is selected to be close to that value.

Then, the end 3 of the bundle 2 can be applied to in the absence of the optical system 24 or placed in the vicinity of the skin in the presence of the optical system 24, and the corresponding image is observed in a step 31. The image can be analyzed in a step 32 by the micro-computer, so as to determine the mean size of the corneocytes, as explained above. In order to determine the mean size, it is possible to determine the number of corneocytes present in the observation field, for example.

Where appropriate, a plurality of images resulting from moving the end 3 and/or the micropositioner 22 can be analyzed in succession, as shown by arrow 33.

The result of the analysis, e.g. a mean size for the corneocytes, can be displayed in a step 34, with at least some of the corresponding information being saved to memory, where appropriate, e.g. the mean size.

Naturally, it is not beyond the ambit of the present invention for the device 4 and the processor system 20 not to form two distinct entities, but rather a single, common entity, the device 4 then being equipped with a keyboard and with a screen, for example.

The invention may make it possible to obtain a mean size for the corneocytes, and this can be useful, e.g. in order to perform a diagnosis, as shown in Figure 7. In this event, the method may include a first step 40 of acquiring one or more images of the surface of the skin, and a step 41 of establishing a diagnosis from information supplied by the apparatus of the invention, e.g. a mean or median size for the corneocytes.

Where appropriate, the information associated with the corneocytes can be combined with other information relating to the person that is the subject of the study.

By way of example, the diagnosis can relate to aging of the skin. By way of example, it is thus possible to inform the person of their position relative to a reference population, e.g. a population within the same age group.

By way of example, it is possible to give the person a score as a function of that person's degree of aging relative to the reference population. It is also possible to propose a treatment, e.g. applying a substance to retard aging of the skin.

The diagnosis may also relate to a pathology of the skin.

The invention may also be used to reveal the effect of a treatment on the skin, as shown in Figure 8.

In this method, in a first step 50, one or more images can be acquired of a skin surface, so as to determine first information associated with the size of the corneocytes that are present, then in a step 51, the skin is treated, and after treatment, in a step 52, one or more images are again acquired of the now treated region.

The images and/or the information corresponding to steps 50 and 52 may be compared in a step 53, so as to reveal, for example, a change that has occurred in the mean or medium size of the corneocytes, and the effect of the treatment.

By way of example, such a method may make it possible to quantify the activity of an agent, or to reveal an action of an agent during treatment.

As shown in Figure 9, the invention may also be used in a method in which one or more images are acquired in first and second steps 60 and 61 that are successive over time.

In a step 62, the data is then processed as a function of the changes that have occurred in at least one parameter associated with the images, so as to establish a prognosis in a step 63. The prognosis may determine the rate at which the mean size of the corneocytes is changing, so as to predict a subsequent state of aging of the skin. By way of example, the user can be informed of the expected state of the skin at different ages. As a function of the prognosis carried out, one or more substances or treatments can be recommended, where appropriate.

As shown in Figure 10, the invention may also be useful in a method in which two acquisitions are carried out in steps 70 and 71 on different regions Z₁ and Z₂ of the skin, e.g. regions exposed in different ways to a treatment or to an environment, and in a step 72, at least some of the information associated with the corresponding images is compared.

By way of example, such a method makes it possible to reveal the effect of a particular environment on the skin, e.g. the effect of exposure to ultraviolet radiation on photo-aging of the skin.

Naturally, the invention is not limited to the embodiments described above.

For example, the invention may apply to reconstructed skin, for example.

The surface observed using the end 3 of the optical-fiber bundle 2 can be the outside surface of the dermis, or even the inside surface of a hair follicle, e.g. when the size of the bundle 2 makes that possible.

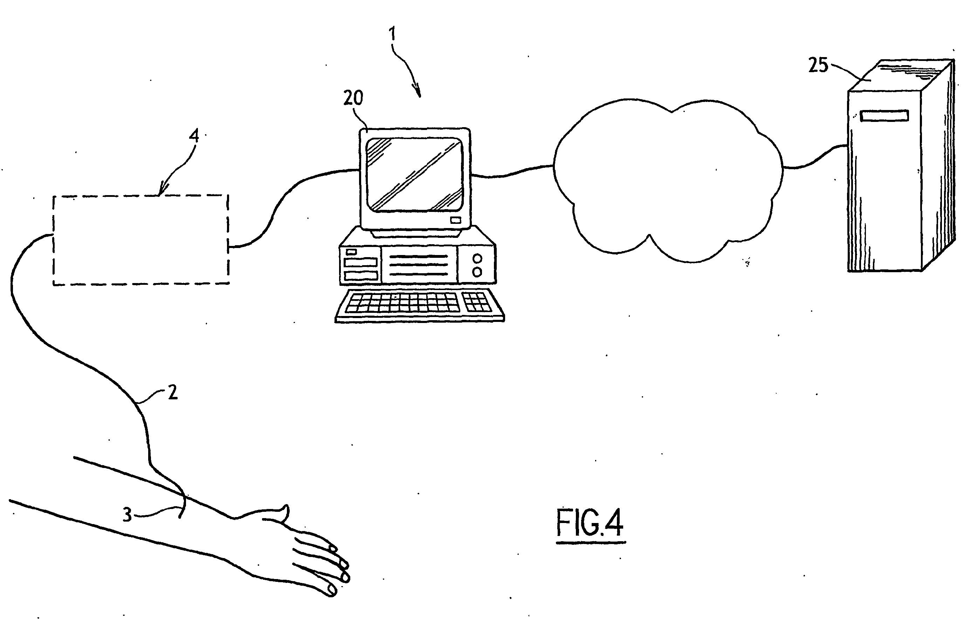

Where appropriate, the image-processor system can be remote, as shown in Figure 4. By way of example, the micro-computer shown in Figure 3 can be connected via a computer and/or telephone network to a server 25 at a processing center, which server sends back information relating, for example, to the size of the corneocytes and/or to a diagnosis, and where appropriate, advice relating to the purchase of a cosmetic. By way of example, the server 25 may be arranged to send information to a goods-shipping center, in order to send a diagnosis accompanied by a cosmetic directly to a consumer. The server 25 may also send information relating to the result of a treatment.

The processor system 20 may also be integrated in the device 4.

The invention can find applications other than in the field of cosmetics, given that certain pathologies of the epidermis affect the size of the corneocytes, e.g. reducing their size in the case of psoriasis.

The invention may also apply to observing keratinous fibers such as hair, for example.

Throughout the description, including in the claims, the expression "comprising a" should be understood as being synonymous with "comprising at least one" unless specified to the contrary.

## Claims

1. A method of observing biological tissue, being skin or keratinous fiber, the method comprising:
· collecting through a first end (3) of a bundle (2) of optical fibers (7) light from a surface of the tissue;
. observing at least one image of the tissue at a second end (5) of the bundle, while injecting light into the second end (5) of the bundle to illuminate the surface of the tissue (23)
wherein light centered on a first wavelength (λ₁) is injected into the fibers and the observation is made at a second wavelength (λ₂) that is different from the first, and
wherein the at least one image is processed so as to determine information associated with corneocytes,and wherein each fiber corresponds to one pixel of the image.

2. A method according to claim 1, wherein the first end (3) of the bundle (2) is put into contact with the surface of the tissue (23).

3. A method according to claim 1, wherein the first end (3) of the bundle (2) is spaced form the surface of the tissue (23) by a distance.

4. A method according to claim 3, wherein light is collected by the first end (3) of the bundle (2) through an optical system (24) comprising at least one lens (17;18).

5. A method according to claim 4, wherein the distance between the optical system (24) and the surface of the tissue (23) ranges from 100 µm to 1 mm.

6. A method according to claim 5, wherein the distance between the optical system (24) and the surface of the tissue (23) ranges from 200 µm to 500 µm.

7. A method according to any one of claims 4 to 6, wherein the at least one lens comprises a magnifying lens.

8. A method according to claim 7, wherein the at least one lens comprises a collimating lens.

9. A method according to any one of claims 4 to 8, wherein the optical system comprises a first lens (17) and second lens (18), light from the surface of the tissue passing through the second lens (18) and then the first lens (17).

10. A method according to the preceding claim, wherein the first lens (17) is a collimating lens and the second lens (18) is a magnifying lens.

11. A method according to claim 9 or 10, wherein the length of the first lens (17) ranges from 4.4 to 6.8 mm.

12. A method according to any one of claims 9 to 11, wherein the length of the second lens (18) ranges from 1.8 to 2.9 mm.

13. A method according to any one of claims 4 to 12, wherein the at least one lens comprises a lens with an index gradient.

14. A method according to the preceding claim, wherein the at least one lens with an index gradient has a g value of the index gradient at 670 nm greater or equal to 0.25 mm⁻¹_{.}

15. A method according to any one of claims 4 to 14, wherein the at least one lens is cylindrical.

16. A method according to claim 15, wherein the at least one lens has an external diameter less than or equal to 2 mm.

17. A method according to claim 1, wherein the spatial resolution of the bundle (2) is better than 5 µm.

18. A method according to the preceding claim, wherein the spatial resolution is better than 4 µm.

19. A method according to the preceding claim, wherein the spatial resolution is better than 3 µm.

20. A method according to the preceding claim, wherein the spatial resolution is better than 1.5 µm.

21. A method according to any preceding claim, wherein the optical fibers (7) have a diameter that is less than or equal to 4 µm.

22. A method according to the preceding claim, wherein the diameter lies in the range from about 2 µm to about 3 µm.

23. A method according to any preceding claim, wherein the bundle (2) comprises not less than 2500 optical fibers.

24. A method according to the preceding claim, wherein the bundle (2) comprises not less than 10,000 optical fibers (7).

25. A method according to any preceding claim, wherein a section occupied by the bundle (2) of optical fibers (7) is greater than or equal to 0.01 mm².

26. A method according to the preceding claim, wherein the section is greater or equal to 0.1 mm².

27. A method according to the preceding claim, wherein the observation is a made *in vivo* observation.

28. A method according to any one of claims 1 to 26, wherein the observation is a made *in vitro* observation.

29. A method according to any preceding claim, wherein the information relates to the number of corneocytes present in the at least one image.

30. A method according to any preceding claim, wherein the information relates to the mean or median size of the corneocytes.

31. A method according to any preceding claim, wherein prior to making the observation, a fluorescent marker is put into contact with the tissue.

32. A method according to the preceding claim, wherein the wavelengths are selected as a function of the fluorescent marker, so as to maximize contrast in the obtained image.

33. A method according to any preceding claim, wherein the at least one image is acquired with an array sensor (16).

34. A method according to the preceding claim, wherein the array sensor (16) is a CCD sensor.

35. A method according to any preceding claim, wherein at least two observations are made at two different locations of the tissue.

36. A method according to the preceding claim, wherein two observations are made at two respective regions of the tissue that have been exposed in different ways to a given environment.

37. A method according to the preceding claim, wherein the two regions have been exposed in different ways to ultraviolet light.

38. A method according to any one of claims 35 to 36, wherein the two regions have been treated differently with a substance.

39. A method according to any preceding claim, wherein information relating to aging of the skin is delivered.

40. A method according to any preceding claims, wherein the surface of the tissue is situated inside a hair follicle.

41. A method according to any preceding claim, wherein the result of an observation is compared with reference data.

42. A method according to any preceding claim, wherein a plurality of observations are made at different locations, and wherein the results of the various observations are processed statistically to obtain a value that is representative of an observed parameter.

43. A method according to the preceding claim, wherein the value is a mean or median value.

44. A method of revealing the effect of a treatment, the method comprising:
· before the treatment, making a first observation by means of the method as defined in any preceding claim;
· after the treatment, making a second observation by means of the method as defined in any preceding claim; and
· comparing the results of the first and second observations.

45. A method according to the preceding claim, wherein information relating to at least one effect of the treatment is determined as a function of the comparison of the results of the first and second observations.

46. A method of predicting changes that will occur in at least one physical and/or biological parameter of some tissue, the method comprising:
· making at least one observation by implementing the method as defined in any one of claims 1 to 43; and
· from the result of the observation, predicting changes that will occur in said parameter.

47. A method of prescribing a cosmetic, the method comprising:
· making at least one observation by implementing the method as defined in any one of claims 1 to 43; and
· from the result of the observation, prescribing at least one cosmetic.

48. Skin-imaging apparatus comprising:
· an image-processor system (20) configured to deliver at least one information associated with observed corneocytes, and **characterised by** · a bundle (2) of optical fibers (7) having a first end (3) for collecting light from a surface of a tissue (23); · an injector system for injecting light into the bundle at a second end (5) thereof; · an analyzer system for analyzing an image formed at the second end (5) of the bundle (2),
· a dichroic mirror (13),
· a light source (10),
· a camera,
. a monochromatic filter or a monochromator associated with the light source,
· a monochromatic filter or a monochromator associated with the camera,
the dichroic mirror (13) reflecting, into the bundle (2) of fibers (7), a fraction of the light emitted by the light source (10), and the camera observing the image that is reflected back via the fibers, after passing through the dichroic mirror,
and each fiber corresponding to one pixel of the image.

49. Apparatus (1) according to the preceding claim,
wherein the resolution of said bundle (2) is sufficient for allowing observation of corneocytes at the surface of the skin.

50. Apparatus (1) according to any one of claims 48 to 49, wherein the first end (3) of the bundle (2) is configured for contacting skin.

51. Apparatus (1) according to any one of claims 48 to 50, wherein the first end (3) of the bundle (2) is configured for observing skin while being spaced from the surface of the tissue by a distance.

52. Apparatus (1) according to any one of claims 48 to 51, further comprising an optical system (24) comprising at least one lens.

53. Apparatus according to claims 51 and 52, wherein the distance between the optical systems (24) and the surface of the tissue (23) ranges from 100 µm to 1 mm.

54. Apparatus according to claim 52 or 53, wherein the at least one lends comprises a magnifying lens.

55. Apparatus according to claim 52, wherein the at least one lens comprises a collimating lens.

56. Apparatus according to any one of claims 52 to 55, wherein the optical system (24) comprises a first lens (17) and second lens (18), light from the surface of the tissue passing through the second lens (18) and then the first lens (17).

57. Apparatus (1) according to the preceding claim, wherein the first lens (17) is a collimating lens and the second lens is a magnifying lens.

58. Apparatus (1) according to any one of claims 56 to 57, wherein the length of the first lens (17) ranges from 4.4 to 6.8 mm.

59. Apparatus (1) according to any one of claims 56 to 58, wherein the length of the second lens (18) ranges from 1.8 to 2.9 mm.

60. Apparatus according to any one of claims 57 to 59, wherein the at least one lens comprises a lens with an index gradient.

61. Apparatus according to the preceding claim, wherein the at least one lens with an index gradient has a g value of the index gradient at 670 nm greater or equal to 0.25 mm⁻¹.

62. Apparatus according to any one of claims 56 to 61, wherein the at least one lens is cylindrical.

63. Apparatus according to the preceding claim, wherein the at least one lens has an external diameter less than or equal to 2 mm.

64. Apparatus according to any one of claims 48 to 63, further including a recorder system enabling images to be stored.

65. Apparatus according to any one of claims 48 to 64, wherein the fibers at the inlet and at the outlet of the bundle have identical positions relative to one another.

66. A kit comprising:
· apparatus as defined in any one of claims 48 to 65; and
· a receptacle containing a fluorescent marker for applying to the skin before it is observed.

## Patentansprüche

1. Ein Verfahren zum Beobachten biologischen Gewebes, das Haut oder keratinhaltige Faser ist, wobei das Verfahren Folgendes umfasst:
- durch das erste Ende (3) eines Bündels (2) von optischen Fasern (7) wird Licht von einer Oberfläche des Gewebes gesammelt;
- wenigstens ein Bild des Gewebes wird an einem zweiten Ende (5) des Bündels beobachtet, während Licht in das zweite Ende (5) des Bündels eingespeist wird, um die Oberfläche des Gewebes (23) zu beleuchten,
wobei Licht, das auf einer ersten Wellenlänge (λ₁) zentriert ist, in die Fasern eingespeist wird und die Beobachtung bei einer zweiten Wellenlänge (λ₂), die von der ersten verschieden ist, gemacht wird, und
wobei das wenigstens eine Bild verarbeitet wird, um Informationen zu bestimmen, die mit Korneozyten verbunden sind, und wobei jede Faser einem Pixel des Bildes entspricht.

2. Ein Verfahren gemäß Anspruch 1, bei dem das erste Ende (3) des Bündels (2) in Kontakt mit der Oberfläche des Gewebes (23) gebracht wird.

3. Ein Verfahren gemäß Anspruch 1, bei dem das erste Ende (3) des Bündels (2) von der Oberfläche des Gewebes (23) durch einen Abstand beabstandet ist.

4. Ein Verfahren gemäß Anspruch 3, bei dem das Licht vom ersten Ende (3) des Bündels (2) durch ein optisches System (24) gesammelt wird, das wenigstens eine Linse (17; 18) umfasst.

5. Ein Verfahren gemäß Anspruch 4, bei dem der Abstand zwischen dem optischen System (24) und der Oberfläche des Gewebes (23) zwischen 100 µm und 1 mm liegt.

6. Ein Verfahren gemäß Anspruch 5, bei dem der Abstand zwischen dem optischen System (24) und der Oberfläche des Gewebes (23) zwischen 200 µm und 500 µm liegt.

7. Ein Verfahren gemäß irgendeinem der Ansprüche 4 bis 6, bei dem die wenigstens eine Linse eine Vergrößerungslinse umfasst.

8. Ein Verfahren gemäß Anspruch 7, bei dem die wenigstens eine Linse eine Kollimatorlinse umfasst.

9. Ein Verfahren gemäß irgendeinem der Ansprüche 4 bis 8, bei dem das optische System eine erste Linse (17) und eine zweite Linse (18) umfasst, wobei Licht von der Oberfläche des Gewebes durch die zweite Linse (18) und dann die erste Linse (17) hindurchgeht.

10. Ein Verfahren gemäß dem vorhergehenden Anspruch, bei dem die erste Linse (17) eine Kollimatorlinse und die zweite Linse (18) eine Vergrößerungslinse ist.

11. Ein Verfahren gemäß Anspruch 9 oder 10, bei dem die Länge der ersten Linse (17) zwischen 4,4 und 6,8 mm liegt.

12. Ein Verfahren gemäß irgendeinem der Ansprüche 9 bis 11, bei dem die Länge der zweiten Linse (18) zwischen 1,8 und 2,9 mm liegt.

13. Ein Verfahren gemäß irgendeinem der Ansprüche 4 bis 12, bei dem die wenigstens eine Linse eine Linse mit einem Indexgradienten umfasst.

14. Ein Verfahren gemäß dem vorhergehenden Anspruch, bei dem die wenigstens eine Linse mit einem Indexgradienten einen g-Wert des Indexgradienten bei 670 nm hat, der größer oder gleich 0,25 mm⁻¹ ist.

15. Ein Verfahren gemäß irgendeinem der Ansprüche 4 bis 14, bei dem die wenigstens eine Linse zylindrisch ist.

16. Ein Verfahren gemäß Anspruch 15, bei dem die wenigstens eine Linse einen äußeren Durchmesser von kleiner oder gleich 2 mm hat.

17. Ein Verfahren gemäß Anspruch 1, bei dem die räumliche Auflösung des Bündels (2) besser als 5 µm ist.

18. Ein Verfahren gemäß dem vorhergehenden Anspruch, bei dem die räumliche Auflösung besser als 4 µm ist.

19. Ein Verfahren gemäß dem vorhergehenden Anspruch, bei dem die räumliche Auflösung besser als 3 µm ist.

20. Ein Verfahren gemäß dem vorhergehenden Anspruch, bei dem die räumliche Auflösung besser als 1,5 µm ist.

21. Ein Verfahren gemäß dem vorhergehenden Anspruch, bei dem die optischen Fasern (7) einen Durchmesser haben, der kleiner oder gleich 4 µm ist.

22. Ein Verfahren gemäß dem vorhergehenden Anspruch, bei dem der Durchmesser in dem Bereich von 2 µm bis etwa 3 µm liegt.

23. Ein Verfahren gemäß dem vorhergehenden Anspruch, bei dem das Bündel (2) nicht weniger als 2500 optische Fasern umfasst.

24. Ein Verfahren gemäß dem vorhergehenden Anspruch, bei dem das Bündel (2) nicht weniger als 10.000 optische Fasern (7) umfasst.

25. Ein Verfahren gemäß dem vorhergehenden Anspruch, bei dem ein Abschnitt, der von dem Bündel (2) optischer Fasern (7) belegt wird, größer oder gleich 0,01 mm² ist.

26. Ein Verfahren gemäß dem vorhergehenden Anspruch, bei dem der Abschnitt größer oder gleich 0,1 mm² ist.

27. Ein Verfahren gemäß dem vorhergehenden Anspruch, bei dem die Beobachtung als Beobachtung in vivo durchgeführt wird.

28. Ein Verfahren gemäß irgendeinem der Ansprüche 1 bis 26, bei dem die Beobachtung als Beobachtung in vitro durchgeführt wird.

29. Ein Verfahren gemäß irgendeinem vorhergehenden Anspruch, bei dem die Information sich auf eine Anzahl Korneozyten bezieht, die in dem wenigstens einen Bild vorhanden sind.

30. Ein Verfahren gemäß irgendeinem vorhergehenden Anspruch, bei dem die Information sich auf die mittlere oder mediane Größe der Korneozyten bezieht.

31. Ein Verfahren gemäß irgendeinem vorhergehenden Anspruch, bei dem vor Durchführung der Beobachtung ein fluoreszenter Marker in Kontakt mit dem Gewebe gebracht wird.

32. Ein Verfahren gemäß dem vorhergehenden Anspruch, bei dem die Wellenlängen als Funktion des fluoreszenten Markers ausgewählt werden, um den Kontrast in dem erhaltenen Bild zu maximieren.

33. Ein Verfahren gemäß irgendeinem vorhergehenden Anspruch, bei dem das wenigstens eine Bild mit einem Arraysensor (16) erhalten wird.

34. Ein Verfahren gemäß dem vorhergehenden Anspruch, bei dem der Arraysensor (16) ein CCD-Sensor ist.

35. Ein Verfahren gemäß irgendeinem vorhergehenden Anspruch, bei dem wenigstens zwei Beobachtungen an zwei verschiedenen Orten des Gewebes durchgeführt werden.

36. Ein Verfahren gemäß dem vorhergehenden Anspruch, bei dem jeweils eine Beobachtung an zwei Regionen des Gewebes durchgeführt wird, die in unterschiedlicher Weise einer gegebenen Umgebung ausgesetzt worden sind.

37. Ein Verfahren gemäß dem vorhergehenden Anspruch, bei dem die zwei Regionen in unterschiedlicher Weise ultravioletter Strahlung ausgesetzt worden sind.

38. Ein Verfahren gemäß irgendeinem der Ansprüche 35 bis 36, bei dem die zwei Regionen unterschiedlich mit einer Substanz behandelt worden sind.

39. Ein Verfahren gemäß irgendeinem vorhergehenden Anspruch, bei dem Informationen geliefert werden, die sich auf das Altern der Haut beziehen.

40. Ein Verfahren gemäß irgendwelchen vorhergehenden Ansprüchen, bei dem die Oberfläche des Gewebes innerhalb eines Haarfollikels gelegen ist.

41. Ein Verfahren gemäß irgendeinem vorhergehenden Anspruch, bei dem das Ergebnis der Beobachtung mit Referenzdaten verglichen wird.

42. Ein Verfahren gemäß irgendeinem vorhergehenden Anspruch, bei dem eine Mehrzahl von Beobachtungen an unterschiedlichen Orten durchgeführt werden und bei dem die Ergebnisse der verschiedenen Beobachtungen statistisch verarbeitet werden, um einen Wert zu erhalten, der repräsentativ für einen beobachteten Parameter ist.

43. Ein Verfahren gemäß dem vorhergehenden Anspruch, bei dem der Wert ein Mittelwert oder Medianwert ist.

44. Ein Verfahren zum Auffinden des Effekts einer Behandlung, wobei das Verfahren Folgendes umfasst:
- vor der Behandlung wird eine erste Beobachtung mittels des Verfahrens, wie es in irgendeinem vorhergehenden Anspruch definiert ist, durchgeführt;
- nach der Behandlung wird eine zweite Beobachtung mittels des Verfahrens, wie es in irgendeinem vorhergehenden Anspruch definiert ist, durchgeführt;
- die Ergebnisse der ersten und zweiten Beobachtung werden verglichen.

45. Ein Verfahren gemäß dem vorhergehenden Anspruch, bei dem Informationen, die sich auf wenigstens einen Effekt der Behandlung beziehen, als Funktion des Vergleichs der Ergebnisse der ersten und zweiten Beobachtung bestimmt werden.

46. Ein Verfahren zum Vorhersagen von Veränderungen, die in wenigstens einem physikalischen und/oder biologischen Parameter irgendeines Gewebes auftreten werden, wobei das Verfahren Folgendes umfasst:
- es wird wenigstens eine Beobachtung durchgeführt, indem das Verfahren, wie es in irgendeinem der Ansprüche 1 bis 43 definiert ist, implementiert wird; und
- aus dem Ergebnis der Beobachtung werden Veränderungen vorhergesagt, die in dem Parameter auftreten werden:

47. Ein Verfahren zum Verschreiben eines Kosmetikums, wobei das Verfahren Folgendes umfasst:
- es wird wenigstens eine Beobachtung durchgeführt, indem das Verfahren, wie es in irgendeinem der Ansprüche 1 bis 43 definiert ist, implementiert wird; und
- aus dem Ergebnis der Beobachtung wird wenigstens ein Kosmetikum verschrieben.

48. Eine Hautabbildungsvorrichtung, die Folgendes umfasst:
- ein Bildverarbeitungssystem (20), das eingerichtet ist, wenigstens eine Information, die mit beobachteten Korneozyten verknüpft ist, zu liefern, und **gekennzeichnet ist durch**
ein Bündel (2) optischer Fasern (7), die ein erstes Ende zum Sammeln von Licht von einer Oberfläche eines Gewebes (23) haben;
ein Einspeisungssystem zum Einspeisen von Licht in das Bündel an einem zweiten Ende (5) davon;
ein Analysatorsystem zum Analysieren eines Bildes, das an einem zweiten Ende (5) des Bündels (2) ausgebildet ist;
- einen dichroitischen Spiegel (13),
- eine Lichtquelle (10);
- eine Kamera;
- ein monochromatisches Filter oder einen Monochromator, das bzw. der der Lichtquelle zugeordnet ist;
- ein monochromatisches Filter oder einen Monochromator, das bzw. der der Kamera zugeordnet ist;
wobei der dichroitische Spiegel (13) einen Bruchteil des Lichts in das Bündel (2) von Fasern (7) reflektiert, das von der Lichtquelle (10) emittiert wird, und die Kamera das Bild beobachtet, das über die Fasern zurück reflektiert wird, nachdem es **durch** den dichroitischen Spiegel gegangen ist, und wobei jede Faser einem Pixel des Bildes entspricht.

49. Vorrichtung (1) gemäß dem vorhergehenden Anspruch, bei der die Auflösung des Bündels (2) ausreicht, um eine Beobachtung von Korneozyten an der Oberfläche der Haut zu gestatten.

50. Vorrichtung (1) gemäß irgendeinem der Ansprüche 48 bis 49, bei der das erste Ende (3) des Bündels (2) eingerichtet ist, die Haut zu berühren.

51. Vorrichtung (1) gemäß irgendeinem der Ansprüche 48 bis 50, bei der das erste Ende (3) des Bündels (2) eingerichtet ist, die Haut zu beobachten, während es von der Oberfläche des Gewebes durch einen Abstand beabstandet ist.

52. Vorrichtung (1) gemäß irgendeinem der Ansprüche 48 bis 51, die ferner ein optisches System (24) umfasst, das wenigstens eine Linse umfasst.

53. Vorrichtung gemäß Ansprüchen 51 und 52, bei der der Abstand zwischen dem optischen System (24) und der Oberfläche des Gewebes (23) zwischen 100 µm und 1 mm liegt.

54. Vorrichtung gemäß irgendeinem der Ansprüche 51 oder 52, bei der die wenigstens eine Linse eine Vergrößerungslinse umfasst.

55. Vorrichtung gemäß Anspruch 52, bei der die wenigstens eine Linse eine Kollimatorlinse umfasst.

56. Vorrichtung gemäß irgendeinem der Ansprüche 52 bis 55, bei der das optische System eine erste Linse (17) und eine zweite Linse (18) umfasst, wobei Licht von der Oberfläche des Gewebes durch die zweite Linse (18) und dann die erste Linse (17) hindurchgeht.

57. Vorrichtung (1) gemäß dem vorhergehenden Anspruch, bei der die erste Linse (17) eine Kollimatorlinse und die zweite Linse (18) eine Vergrößerungslinse ist.

58. Vorrichtung (1) gemäß irgendeinem der Ansprüche 56 bis 57, bei der die Länge der ersten Linse (17) zwischen 4,4 und 6,8 mm liegt.

59. Vorrichtung (1) gemäß irgendeinem der Ansprüche 56 bis 58, bei der die Länge der zweiten Linse (18) zwischen 1,8 und 2,9 mm liegt.

60. Vorrichtung gemäß irgendeinem der Ansprüche 57 bis 59, bei der die wenigstens eine Linse eine Linse mit einem Indexgradienten umfasst.

61. Vorrichtung gemäß dem vorhergehenden Anspruch, bei der die wenigstens eine Linse mit einem Indexgradienten einen g-Wert des Indexgradienten bei 670 nm hat, der größer oder gleich 0,25 mm⁻¹ ist.

62. Vorrichtung gemäß irgendeinem der Ansprüche 56 bis 61, bei der die wenigstens eine Linse zylindrisch ist.

63. Vorrichtung gemäß dem vorhergehenden Anspruch, bei der die wenigstens eine Linse einen äußeren Durchmesser von kleiner oder gleich 2 mm hat.

64. Vorrichtung gemäß irgendeinem der Ansprüche 48 bis 63, die ferner ein Aufnahmesystem beinhaltet, das das Speichern vorn Bildern ermöglicht.

65. Vorrichtung gemäß irgendeinem der Ansprüche 48 bis 64, bei der die Fasern am Einlass und am Auslass des Bündels identische Positionen relativ zu einander haben.

66. Ein Ausrüstungssatz, der Folgendes umfasst:
- eine Vorrichtung, wie in irgendeinem der Ansprüche 48 bis 65 definiert; und
- ein Behältnis, das einen fluoreszenten Marker zur Anwendung auf der Haut vor deren Beobachtung beinhaltet.

## Revendications

1. Procédé d'observation d'un tissu biologique, le tissu étant la peau ou une fibre kératinique, comportant les étapes suivantes :
- recevoir à travers une première extrémité (3) des fibres d'un faisceau (2) de fibres optiques (7) la lumière venant d'une surface du tissu,
- observer au moins une image du tissu à une deuxième extrémité (5) des fibres du faisceau, alors qu'on injecte de la lumière à travers la seconde extrémité (5) du faisceau pour éclairer la surface du tissu (23),
une lumière centrée sur une première longueur d'onde (λ₁) étant injectée dans les fibres et l'observation s'effectuant à une deuxième longueur d'onde (λ₂) différente de la première, ladite au moins une image étant traitée pour déterminer une information liée aux coméocytes, et chaque fibre correspondant à un pixel de l'image.

2. Procédé selon la revendication 1, **caractérisé par le fait que** la première extrémité (3) du faisceau (2) est mise en contact avec la surface du tissu (23).

3. Procédé selon la revendication 1, **caractérisé par le fait que** la première extrémité (3) du faisceau (2) est à une distance non nulle de la surface du tissu (23).

4. Procédé selon la revendication 3, **caractérisé par le fait que** la lumière est recueillie à la première extrémité (3) du faisceau (2) à travers un système optique (24) comprenant au moins une lentille (17,18).

5. Procédé selon la revendication 4, **caractérisé par le fait que** la distance entre les systèmes optiques (24) et la surface du tissu (23) est comprise entre 100µm et 1mm.

6. Procédé selon la revendication 5, **caractérisé par le fait que** la distance entre le système optique (24) et la surface du tissu (23) est comprise entre 200µm et 500µm.

7. Procédé selon l'une quelconque des revendications 4 à 6, **caractérisé par le fait que** la au moins une lentille comporte une lentille grossissante.

8. Procédé selon la revendication 7, **caractérisé par le fait que** la au moins une lentille comporte une lentille collimatrice.

9. Procédé selon l'une quelconque des revendications 4 à 8, **caractérisé par le fait que** le système optique comporte une première lentille (17) et une seconde lentille (18), la lumière réfléchie par la surface du tissu traversant la seconde lentille (18) puis la première lentille (17).

10. Procédé selon la revendication précédente, **caractérisé par le fait que** la première lentille (17) est une lentille collimatrice et que la seconde lentille (18) est une lentille grossissante.

11. Procédé selon la revendication 9 ou 10, **caractérisé par le fait que** la longueur de la première lentille (17) est comprise entre 4, 4 mm et 6,8 mm.

12. Procédé selon l'une quelconque des revendications 9 à 11, **caractérisé par le fait que** la longueur de la seconde lentille (18) est comprise entre 1,8 et 2,9mm.

13. Procédé selon l'une quelconque des revendications 4 à 12, **caractérisé par le fait que** la au moins une lentille comporte une lentille à gradient d'indice.

14. Procédé selon la revendication précédente, **caractérisé par le fait que** la au moins une lentille à gradient d'indice avec une valeur g du gradient d'indice supérieure ou égale à 0,25mm⁻¹ à 670nm.

15. Procédé selon l'une quelconque des revendications 4 à 14, **caractérisé par le fait que** la au moins une lentille est cylindrique.

16. Procédé selon la revendication 15, **caractérisé par le fait que** la au moins une lentille a un diamètre externe inférieur ou égal à 2mm.

17. Procédé selon la revendication 1, **caractérisé par le fait que** la résolution du faisceau est meilleure que 5 µm.

18. Procédé selon la revendication précédente **caractérisé par le fait que** la résolution du faisceau est meilleure que 4µm.

19. Procédé selon la revendication précédente **caractérisé par le fait que** la résolution du faisceau est meilleure que 3µm.

20. Procédé selon la revendication précédente **caractérisé par le fait que** la résolution du faisceau est meilleure que 1,5µm.

21. Procédé selon l'une quelconque des revendications précédentes, **caractérisé par le fait que** les fibres optiques (7) présentent un diamètre inférieur ou égal à 4 µm.

22. Procédé selon la revendication précédente, **caractérisé par le fait que** le diamètre est compris entre environ 2µm et environ 3µm.

23. Procédé selon l'une quelconque des revendications précédentes, **caractérisé par le fait que** le nombre de fibres optiques (7) du faisceau est supérieur ou égal à 2 500.

24. Procédé selon la revendication 4, **caractérisé par le fait que** le nombre de fibres optiques (7) du faisceau (2) est supérieur ou égal à 10 000.

25. Procédé selon l'une quelconque des revendications précédentes, **caractérisé par le fait que** la section occupée par le faisceau (2) de fibres optiques (7) est supérieure ou égale à 0,01 mm².

26. Procédé selon les revendications précédentes, **caractérisé par le fait que** la section est supérieure ou égale à 0,1 mm².

27. Procédé selon la revendication précédente, **caractérisé par le fait que** l'observation s'effectue *in vivo*.

28. Procédé selon l'une quelconque des revendications 1 à 26, **caractérisé par le fait que** l'observation s'effectue *in vitro*.

29. Procédé selon l'une quelconque des revendications précédentes, **caractérisé par le fait que** l'information porte notamment sur le nombre de cornéocytes présents sur l'image.

30. Procédé selon l'une quelconque des revendications précédentes, **caractérisé par le fait que** l'information porte notamment sur la taille moyenne ou médiane des cornéocytes.

31. Procédé selon l'une quelconque des revendications précédentes, **caractérisé par le fait que** préalablement à l'observation un marqueur fluorescent est mis en contact du tissu.

32. Procédé selon la revendication précédente, **caractérisé par le fait que** les longueurs d'ondes (λ₁) et (λ₂) sont choisies en fonction du marqueur fluorescent de manière à maximiser le contraste de l'image obtenue.

33. Procédé selon l'une quelconque des revendications précédentes, **caractérisé par le fait que** l'image est acquise avec un capteur matriciel.

34. Procédé selon la revendication précédente **caractérisé par le fait que** le capteur matriciel est un capteur CCD.

35. Procédé selon l'une quelconque des revendications précédentes, **caractérisé par le fait que** l'on procède à au moins deux observations en deux endroits différents du tissu.

36. Procédé selon la revendication précédente, **caractérisé par le fait que** l'on procède à deux observations respectivement en deux régions du tissu exposées différemment à un environnement donné.

37. Procédé selon la revendication précédente, **caractérisé par le fait que** les deux régions ont été exposées différemment à la lumière ultraviolette.

38. Procédé selon l'une quelconque des revendications 35 à 36, **caractérisé par le fait que** les deux régions ont été traitées différemment par un produit.

39. Procédé selon l'une quelconque des revendications précédentes, **caractérisé par le fait que** l'on délivre une information concernant le vieillissement cutané.

40. Procédé selon l'une quelconque des revendications précédentes, **caractérisé par le fait que** la surface du tissu est située à l'intérieur d'un follicule pileux.

41. Procédé selon l'une quelconque des revendications précédentes, **caractérisé par le fait que** l'on compare le résultat de l'observation avec des données de référence.

42. Procédé selon l'une quelconque des revendications précédentes, **caractérisé par le fait que** l'on procède à plusieurs observations en des endroits différents et **par le fait que** l'on traite statistiquement les résultats des diverses observations pour obtenir une valeur représentative d'un paramètre observé.

43. Procédé selon la revendication précédente, **caractérisé par le fait que** la valeur représentative est une valeur moyenne ou médiane.

44. Procédé pour mettre en évidence l'effet d'un traitement non thérapeutique, comportant les étapes suivantes :
- effectuer avant le traitement une première observation au moyen d'un procédé tel que défini dans l'une quelconque des revendications précédentes,
- effectuer après le traitement une deuxième observation au moyen du procédé tel que défini dans l'une quelconque des revendications précédentes, et
- comparer les résultats des première et deuxième observations.

45. Procédé selon la revendication précédente, **caractérisé par le fait que** l'information relative à au moins un effet du traitement est déterminée en fonction de la comparaison des résultats des premières et deuxièmes observations.

46. Procédé pour prédire un état ultérieur d'au moins un paramètre physique et/ou biologique d'un tissu, comportant les étapes suivantes :
- effectuer au moins une observation en mettant en oeuvre le procédé défini dans l'une quelconque des revendications 1 à 43 ; et ,
- prédire en fonction du résultat de l'observation les changements qui se produiront pour ledit paramètre.

47. Procédé de prescription d'un produit cosmétique, comportant les étapes suivantes :
- effectuer au moins une observation en mettant en oeuvre le procédé défini dans l'une quelconque des revendications 1 à 43 ; et
- à partir du résultat de l'observation, prescrire au moins un produit cosmétique.

48. Dispositif d'imagerie de la peau, comportant :
- un système (20) de traitement d'image permettant de délivrer au moins une information liée aux cornéocytes observés,
**caractérisé par le fait qu'**il comporte également :
- un faisceau (2) de fibres optiques (7) comportant une première extrémité (3) adaptée à recueillir de la lumière provenant d'une surface d'un tissu (23) ;
- un système d'injection de lumière dans une seconde extrémité (5) du faisceau ;
- un système d'analyse d'une image formée à la deuxième extrémité (5) du faisceau (2),
- un miroir dichroïque (13),
- une source lumineuse (10),
- une caméra (16),
- un filtre monochromatique (11) ou un monochromateur associé à la source lumineuse (10),
- un filtre monochromatique (15) ou un monochromateur associé à la caméra (16),
le miroir dichroïque (13) renvoyant une partie de la lumière émise par la source lumineuse (10) dans le faisceau (2) de fibres (7) et la caméra observant l'image renvoyée par les fibres après la traversée du miroir dichroïque,
et chaque fibre correspondant à un pixel de l'image.

49. Dispositif (1) selon la revendication précédente, **caractérisé par le fait que** la résolution du faisceau (2) est suffisante pour permettre l'observation des cornéocytes à la surface de la peau.

50. Dispositif (1) selon l'une quelconque des revendications 48 à 49, **caractérisé par le fait que** la première extrémité (3) du faisceau (2) est agencée pour contacter la peau.

51. Dispositif (1) selon l'une quelconque des revendications 48 à 50, **caractérisé par le fait que** la première extrémité (3) du faisceau (2) est agencée pour observer la peau alors qu'il est espacé de la surface du tissu par une distance.

52. Dispositif (1) selon l'une quelconque des revendications 48 à 51, **caractérisé par le fait qu'**il comporte également un système optique (24) comprenant au moins une lentille.

53. Dispositif selon les revendications 51 et 52, **caractérisé par le fait que** la distance entre le système optique(24) et la surface du tissu (23) est comprise entre 100µm et 1mm.

54. Dispositif selon la revendication 52 ou 53, **caractérisé par le fait que** la au moins une lentille comporte une lentille grossissante.

55. Appareil selon la revendication 52, **caractérisé par le fait que** la au moins une lentille comporte une lentille collimatrice.

56. Dispositif selon l'une des revendications 52 à 55, **caractérisé par le fait que** le système optique (24) comporte une première lentille (17) et une seconde lentille (18), la lumière réfléchie par la lumière du tissu traversant la seconde lentille (18) puis la première lentille (17).

57. Dispositif (1) selon la revendication précédente, **caractérisé par le fait que** la première lentille (17) est une lentille collimatrice et la seconde lentille est une lentille grossissante.

58. Dispositif (1) selon l'une quelconque des revendications 56 à 57, **caractérisé par le fait que** la longueur de la première lentille (17) est comprise entre 4,4 et 6,8mm.

59. Dispositif (1) selon l'une quelconque des revendications 56 à 57, **caractérisé par le fait que** la longueur de la seconde lentille (18) est comprise entre 1,8 et 2,9mm.

60. Dispositif selon l'une quelconque des revendications 57 à 59, **caractérisé par le fait que** la au moins une lentille comporte une lentille à gradient d'indice.

61. Dispositif selon la revendication précédente, **caractérisé par le fait que** la au moins une lentille à gradient d'indice avec une valeur g du gradient d'indice supérieure ou égale à 0,25mm⁻¹ à 670nm.

62. Dispositif selon l'une quelconque des revendications 56 à 61, **caractérisé par le fait que** là au moins une lentille est cylindrique.

63. Dispositif selon la revendication précédente, **caractérisé par le fait que** la au moins une lentille a un diamètre externe inférieur ou égal à 2mm.

64. Dispositif selon l'une quelconque des revendications 48 à 63, **caractérisé par le fait qu'**il comporte un système d'enregistrement permettant de mémoriser des images.

65. Dispositif selon l'une quelconque des revendications 48 à 64, **caractérisé par le fait que** la position relative des fibres entre elles est la même à l'entrée et à la sortie du faisceau.

66. Kit comportant :
- un dispositif tel que défini dans l'une quelconque des revendications 48 à 65,
- un récipient contenant un marqueur fluorescent à appliquer sur la peau avant l'observation.
